# EUROPÄISCHE PATENTANMELDUNG

(11) **EP 1 431 279 A2**
(43) Veröffentlichungstag der Anmeldung: **23.06.2004**
(21) Anmeldenummer: 03026981.5
(22) Anmeldetag: 26.11.2003
(51) Int. Cl.: C07C 255/36, C07C 69/017, C07C 215/50, A61K 31/275, A61P 9/00

(54) **Fluorhaltige Aromaten**

(30) Priorität: 09.12.2002 DE 10257356
(71) Anmelder: Bayer Chemicals AG, 51368 Leverkusen (DE)
(72) Erfinder: Peilstöcker, Karen, Dr., 51061 Köln (DE); Marhold, Albrecht, Dr., 51373 Leverkusen (DE); Pleschke, Axel, Dr., 51069 Köln (DE); Giffels, Guido, Dr., 53177 Bonn (DE)

(57) **Zusammenfassung**

Die vorliegende Erfindung betrifft fluorhaltige Aromaten, ein Verfahren zu deren Herstellung sowie die Anwendung der fluorhaltigen Aromaten zur Herstellung von Wirkstoffen insbesondere in Arzneimitteln und Agrochemikalien.

## Beschreibung

Die vorliegende Erfindung betrifft fluorhaltige Aromaten, ein Verfahren zu deren Herstellung sowie die Anwendung der fluorhaltigen Aromaten zur Herstellung von Wirkstoffen insbesondere in Arzneimitteln und Agrochemikalien.

Fluorhaltige Aromaten, wie insbesondere fluorhaltige Phenole und fluorhaltige Phenolether, sind wertvolle Ausgangsprodukte für die Herstellung von Wirkstoffen in Arzneimitteln und Agrochemikalien, da die Fluor- oder fluorhaltigen Substituenten die Lipophilie und damit die Membrangängigkeit des gesamten Wirkstoffmoleküls erhöhen. So eignen sich beispielsweise substituierte, fluorhaltige Methoxyarylacetonitrile besonders als Ausgangsprodukt zur Herstellung von Arzneimitteln, die zur Behandlung von Herz-Kreislauf-Erkrankungen eingesetzt werden (siehe auch WO-A 01/19780).

Die Herstellung von 5-Fluor-2-hydroxyphenylacetonitril kann beispielsweise ausgehend von 5-Fluor-2-hydroxybenzaldehyd durch eine vierstufige Synthesesequenz hergestellt werden (WO-A 01/19780, S. 81 ff).

Nachteilig an dieser Methode ist, dass die Synthesesequenz sehr lang ist und von einem Edukt ausgeht, das seinerseits entweder nur umständlich oder in sehr geringen Ausbeuten durch Formylierung von p-Fluor-phenol hergestellt werden konnte (Suzuki et al., Chem. Pharm. Bull. 1963, 31(5), 1751-1753) und somit sehr teuer ist.

Es bestand daher das Bedürfnis, ein Verfahren bereitzustellen, das die Herstellung von fluorhaltigen Phenylacetonitrilen in guten Ausbeuten und in einfacher Weise ermöglicht.

Es wurde nun ein Verfahren zur Herstellung von Verbindungen der Formel (I) gefunden, 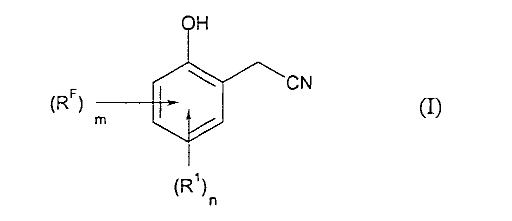 in der
- R¹: jeweils unabhängig für C₁-C₁₂-Alkyl, freies oder geschütztes Formyl, Chlor oder Brom oder für Reste der Formeln (IIa) oder (IIb) steht,

A-B-D-E (IIa)

A-E (IIb)

in denen unabhängig voneinander
A fehlt oder für einen C₁-C₈-Alkylenrest steht und
B fehlt oder für Sauerstoff, Schwefel oder NR² steht, wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
- n: für eine ganze Zahl von 0 bis 4-m steht und
- R^{F}: für Fluor, C₁-C₁₂-Fluoralkyl, -O(C₁-C₁₂-Fluoralkyl) oder -S(C₁-C₁₂-Fluoralkyl) steht und
- m: für eine ganze Zahl von 1 bis 3 steht,
das dadurch gekennzeichnet ist, dass
a) Verbindungen der Formel (II) 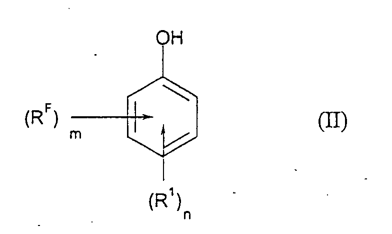 in der R¹ und R^{F}, sowie n und m die vorstehend genannte Bedeutung besitzen,
   - in Gegenwart von Formaldehyd und
   - in Gegenwart von sekundären Aminen der Formel (III)

      HNR³R⁴ (III)

      in der R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht
   zu Verbindungen der Formel (IV) umgesetzt werden, 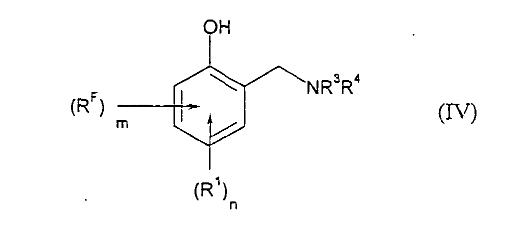 in der R¹, R³, R⁴ und R^{F} sowie m und n die vorstehend genannte Bedeutung besitzen und
b) die Verbindungen der Formel (IV) durch Umsetzung mit Verbindungen der Formel (V)

   R⁵CO-O-OCR⁵ (V)

   in der die Reste R⁵ jeweils unabhängig voneinander für Wasserstoff, C₁-C₁₂-Alkyl, C₂-C₁₂-Alkenyl, C₅-C₁₄-Aryl oder C₆-C₁₅-Arylalkyl stehen,
   in Verbindungen der Formel (VI) überführt werden 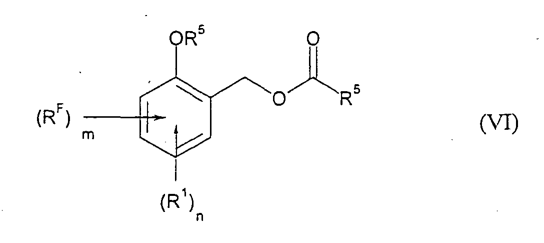 und
c) die Verbindungen der Formel (VI) durch Umsetzung mit Cyanid in Verbindungen der Formel (I) überführt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Alkyl** beziehungsweise Alkylen beziehungsweise Alkoxy beziehungsweise Alkenyl bedeutet jeweils unabhängig einen geradkettigen, cyclischen, verzweigten oder unverzweigten Alkyl- beziehungsweise Alkylen- beziehungsweise Alkoxy- beziehungsweise Alkenyl-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, n-Nonyl, n-Decyl und n-Dodecyl.

C₁-C₈-Alkoxy steht beispielsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy.

**Fluoralkyl** steht jeweils unabhängig einen geradkettigen, cyclischen, verzweigten oder unverzweigten Alkylrest, der durch mindestens ein Fluoratom und gegebenenfalls weiter durch Chloratome und/oder Bromatome substituiert ist.

Beispielsweise steht C₁-C₁₂-Polyfluoralkyl für Trifluormethyl, Chlorfluormethyl, Difluormethyl, Difluorchlormethyl, 1,1,2,2-Tetrafluor-1-ethyl, 2-Chlor-2,1,1-trifluor-1-ethyl, 2,2,2-Trifluorethyl, Pentafluorethyl, 1,1-Dichlor-2,2,2-trifluorethyl, Heptafluorisopropyl, n-Nonafluorbutyl, Perfluorcyclopentyl, Perfluorcyclohexyl und Perfluordodecyl.

**Aryl** bedeutet jeweils unabhängig einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 14 Gerüstkohlenstoffatomen.

Beispiele für carbocyclische aromatische Reste mit 6 bis 14 Gerüstkohlenstoffatomen sind Phenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuranyl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-C₁₂-Alkyl, C₁-C₁₂-Perfluoralkyl, COO(C₁-C₈-Alkyl), CON(C₁-C₈-Alkyl)₂, COO(C₁-C₈-Arylalkyl), COO(C₄-C₁₄-Aryl), CO(C₁-C₈-Alkyl), C₅-C₁₅-Arylalkyl oder Tri(C₁-C₆-alkyl)siloxyl.

**Arylalkyl** bedeutet jeweils unabhängig einen geradkettigen, cyclischen, verzweigten oder unverzweigten Alkyl-Rest, der einfach, mehrfach oder vollständig durch Aryl-Reste gemäß obiger Defmition substituiert sein kann.

C₆-C₁₅-Arylalkyl steht beispielsweise und bevorzugt für Benzyl.

Im Folgenden werden die bevorzugten Substitutionsmuster für Verbindungen der Formeln (I) bis (VI) definiert:
- R¹: steht bevorzugt jeweils unabhängig für C₁-C₄-Alkyl, freies oder geschütztes Formyl oder Chlor, besonders bevorzugt für Methyl.
- n: steht bevorzugt für 0 oder 1, besonders bevorzugt für 0.
- R^{F}: steht bevorzugt für Fluor, C₁-C₄-Fluoralkyl, -O(C₁-C₄-Fluoralkyl) oder -S(C₁-C₄-Fluoralkyl), besonders bevorzugt für Trifluormethyl, Trifluormethylthio, Trifluormethoxy, Chlorfluormethyl, Chlorfluormethylthio, Chlorfluormethoxy, Difluormethoxy, Difluormethyl, Difluormethylthio, Difluormethoxy, Difluorchlormethyl, Difluorchlormethylthio, Difluorchlormethoxy, 1,1,2,2-Tetrafluor-1-ethyl, 1,1,2,2-Tetrafluor-1-ethylthio, 1,1,2,2-Tetrafluor-1-ethoxy, 2-Chlor-2,1,1-trifluor-1-ethyl, 2-Chlor-2,1,1-trifluor-1-ethylthio, 2-Chlor-2,1,1-trifluor-1-ethoxy, 2,2,2-Trifluorethyl, 2,2,2-Trifluorethylthio, 2,2,2-Trifluorethoxy, Pentafluorethyl, Pentafluorethylthio, Pentafluorethoxy, 1,1-Dichlor-2,2,2-trifluorethyl, 1,1-Dichlor-2,2,2-trifluorethylthio, 1,1-Dichlor-2,2,2-trifluorethoxy, Heptafluorisopropyl, n-Nonafluorbutyl, Perfluorcyclopentyl, Perfluorcyclohexyl und Perfluordodecyl.
- m: steht bevorzugt für den Fall, dass alle R^{F} für Fluor stehen für eine ganze Zahl von 1 bis 3, ansonsten für eins oder zwei, bevorzugt für eins.
- R³ und R⁴: stehen bevorzugt jeweils für einen identischen C₁-C₈-Alkylrest, besonders bevorzugt identisch für Methyl oder Ethyl.
- R⁵: steht bevorzugt jeweils identisch für Wasserstoff, C₁-C₁₂-Alkyl, C₂-C₁₂-Alkenyl, C₅-C₁₄-Aryl oder C₆-C₁₅-Arylalkyl, besonders bevorzugt für Methyl.

Gemäß Schritt a) werden die Verbindungen der Formel (II) in Gegenwart von Formaldehyd und in Gegenwart von sekundären Aminen der Formel (III) in Verbindungen der Formel (IV) überführt.

Das molare Verhältnis von Formaldehyd zu Verbindungen der Formel (II) kann beispielsweise 0,8 bis 10 betragen, bevorzugt 1,0 bis 10 und besonders bevorzugt 1,2 bis 3,6.

Das molare Verhältnis von sekundären Aminen der Formel (III) zu Verbindungen der Formel (II) kann beispielsweise 0,8 bis 10 betragen, bevorzugt 1,0 bis 1.0 und besonders bevorzugt 1,05 bis 3,15.

Formaldehyd kann dabei beispielsweise als Paraformaldehyd und oder in Form einer wässrigen Lösung eingesetzt werden, vorzugsweise in Form einer 32 bis 40 gew.-%igen Lösung.

Die sekundären Amine der Formel (III) können beispielsweise in Substanz oder sofern möglich in Form wässriger Lösungen eingesetzt werden. Besonders bevorzugt wird Dimethylamin in Form einer wässrigen Lösung eingesetzt.

Die Reaktionstemperatur kann beispielsweise -20°C bis 120°C betragen, bevorzugt -10 bis 40°C und besonders bevorzugt -5 bis 10°C.

Der Reaktionsdruck kann beispielsweise 0,5 bis 100 bar betragen, Umgebungsdruck ist bevorzugt.

Die Reaktionsdauer kann 10 min bis 72 Stunden betragen, bevorzugt sind 3 Stunden bis 24 Stunden.

Zur Umsetzung der Verbindungen der Formel (II) geht man beispielsweise so vor, dass die Verbindungen der Formel (II) und die sekundären Amine der Formel (III) vorgelegt werden und anschließend die Zugabe des Formaldehyds erfolgt.

Die Aufarbeitung der Verbindungen der Formel (IV) kann in an sich bekannter Weise durch Extraktion und anschließende Destillation oder bei 30°C festen Verbindungen der Formel (IV) durch Umkristallisation erfolgen.

Die Verbindungen der Formel (IV) sind als wichtige Intermediate von der Erfindung ebenfalls umfasst, wobei jedoch 2-Hydroxy-5-fluor-N,N-dimethylbenzylamin ausgenommen ist. Für die Vorzugsbereiche gelten die vorstehend gemachten Angaben analog.

Als besonders bevorzugte Einzelverbindungen der Formel (IV) seien genannt:
4,5-Difluor-2-hydroxy-N,N-dimethylbenzylamin, 2-Hydroxy-5-(trifluormethoxy)-N,N-dimethylbenzylamin, 6-Hydroxy-2,3,4-trifluor-N,N-dimethylbenzylamin und 2-Hydroxy-4-(trifluormethyl)-N,N-dimethylbenzylamin.

Die für Schritt a) benötigten Ausgangsverbindungen der Formel (II) sind literaturbekannt oder analog zur Literatur synthetisierbar.

Vorzugsweise und ebenfalls erfindungsgemäß stellt man beispielsweise Verbindungen der Formel (Ia) 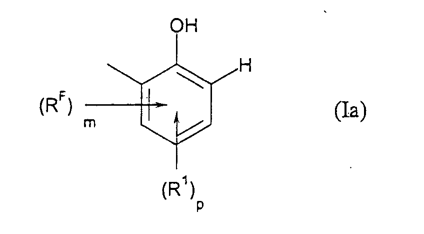 in der R¹, R^{F} und m die vorstehend genannte Bedeutung und Vorzugsbereiche besitzen und
p für eine ganze Zahl zwischen 0 und 3-m steht,
dadurch her, dass Verbindungen der Formel (IVa) 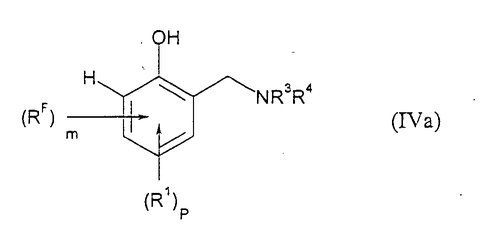 in der R¹, R³, R⁴ und R^{F}, sowie p und m die vorstehend genannte Bedeutung und Vorzugsbereiche besitzen, reduziert.

Die Reduktion kann dabei vorteilhafterweise in Gegenwart von Wasserstoff und Hydrierkatalysator durchgeführt werden.

Bevorzugte Hydrierkatalysatoren sind beispielsweise Metalle oder Metallverbindungen wie Salze oder Komplexe von Nickel, Palladium, Platin, Cobalt, Rhodium, Iridium und Ruthenium, wobei Metalle wie Nickel oder Palladium bevorzugt sind. Vorzugsweise werden Metalle in fein verteilter Form wie zum Beispiel als Raney-Metall oder auf ein Trägermaterial aufgebracht verwendet.

Besonders bevorzugt wird die Reduktion mit Wasserstoff und Raney-Nickel und/oder Palladium auf Kohle durchgeführt.

Die Reduktion kann beispielsweise bei einer Reaktionstemperatur von 20°C bis 200°C, bevorzugt 50 bis 180°C und besonders bevorzugt 80 bis 150°C durchgeführt werden.

Der Wasserstoffpartialdruck kann bei der Reduktion kann beispielsweise 0,1 bis 180 bar betragen, bevorzugt 10 bis 150 bar und besonders bevorzugt 40 bis 120 bar.

Gegebenenfalls und vorzugsweise kann die Reduktion in Gegenwart von Lösungsmittel durchgeführt werden, sofern sie im Wesentlichen unter den gewählten Reduktionsbedingungen inert sind.

Geeignete Lösungsmittel sind beispielsweise aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Alkohole wie beispielsweise Methanol, Ethanol und iso-Propanol, Carbonsäuren wie beispielsweise Essigsäure oder Mischungen von Lösungsmitteln.

Die Reaktionsdauer bei der Reduktion kann 10 min bis 200 Stunden betragen, bevorzugt sind 5 bis 100 Stunden.

In einer besonders bevorzugten Ausführungsform wird die Reduktion in Gegenwart von Palladium auf Aktivkohle und in Gegenwart von Essigsäure bei einem Wasserstoffpartialdruck von 40 bis 120 bar durchgeführt.

Die Verbindungen der Formel (IVa) sind als Teilmenge der Verbindungen der Formel (IV) ebenfalls von der Erfindung umfasst.

Gemäß Schritt b) werden die Verbindungen der Formel (IV) mit Verbindungen der Formel (V) in Verbindungen der Formel (VI) überführt.

Das molare Verhältnis von Verbindungen der Formel (V) zu Verbindungen der Formel (IV) kann beispielsweise 1,5 bis 10 betragen, bevorzugt 2 bis 5 und besonders bevorzugt 2 bis 4.

Die Reaktionstemperatur kann beispielsweise 0°C bis 200°C betragen, bevorzugt 50 bis 150°C und besonders bevorzugt 70 bis 120°C.

Der Reaktionsdruck kann beispielsweise 0,5 bis 100 bar betragen, Umgebungsdruck ist bevorzugt.

Die Reaktionsdauer kann 10 min bis 72 Stunden betragen, bevorzugt sind 3 Stunden bis 8 Stunden.

Gegebenenfalls kann die Umsetzung gemäß Schritt b) in Gegenwart eines aprotischen organischen Lösungsmittels durchgeführt werden.

Aprotisch heißt in Rahmen der Erfindung, dass das organische Lösungsmittel keine Protonen aufweist, die bezogen auf eine wässrige Vergleichsskala bei 25°C einen pKs-Wert von unter 23 besitzen.

Geeignete organische Lösungsmittel sind beispielsweise aliphatische oder aromatische, gegebenenfalls halogenierte Kohlenwasserstoffe wie beispielsweise Benzinfraktionen, Benzol, Toluol, Xylol, Chlorbenzol, Dichlorbenzol, Trichlorbenzole, Chlorbenzotrifluoride, Petrolether, Hexan, Cyclohexan, Dichlormethan, Chloroform oder Tetrachlorkohlenstoff, Ether wie beispielsweise Diethylether, Diisopropylether oder tert.-Butylmethylether.

Die Aufarbeitung der Verbindungen der Formel (VI) kann in an sich bekannter Weise durch Extraktion und anschließende Destillation oder bei 30°C festen Verbindungen der Formel (VI) durch Umkristallisation erfolgen.

Die Verbindungen der Formel (VI) sind als wichtige Intermediate von der Erfindung ebenfalls umfasst, wobei jedoch 2-Acetoxy-5-fluor-benzylacetat (Synthetic Communications, 2000, 30(3), 397-405) ausgenommen ist. Für die Vorzugsbereiche gelten die vorstehend gemachten Angaben analog.

Als besonders bevorzugte Einzelverbindungen der Formel (VI) seien genannt:
2-Acetoxy-4,5-difluorbenzylacetat, 2-Acetoxy-5-(trifluormethoxy)benzylacetat, 6-Acetoxy-2,3,4-trifluorbenzylacetat und 2-Acetoxy-4-trifluormethylbenzylacetat.

Gemäß Schritt c) erfolgt die Umsetzung von Verbindungen der Formel (VI) mit Cyanid zu Verbindungen der Formel (I).

Als Cyanidquelle können beispielsweise Alkalimetallcyanide wie insbesondere Natriumcyanid und Kaliumcyanid dienen.

Das molare Verhältnis von Cyanid zu Verbindungen der Formel (VI) kann beispielsweise 0,8:1 bis 10:1 betragen, bevorzugt 2:1 bis 5:1 und besonders bevorzugt 2,3:1 bis 2,7:1.

Die Reaktionstemperatur kann beispielsweise -20°C bis 100°C betragen, bevorzugt 20 bis 65°C und besonders bevorzugt 20 bis 50°C.

Als Lösungsmittel in Schritt c) eignen sich insbesondere Ether, wie beispielsweise Diethylether, Diisopropylether, Dioxan, Tetrahydrofuran oder Ethylenglykoldimethyl- oder -diethylether; Ketone, wie beispielsweise Aceton, Butanon oder Methyl-isobutyl-keton;

Nitrile, wie beispielsweise Acetonitril, Propionitril oder Benzonitril; Amide, wie beispielsweise N,N-Dimethylformamid, N,N-Dimethylacetamid, N-Methylformanilid, N-Methylpyrrolidon oder Hexamethylphosphorsäuretriamid; Ester, wie beispielsweise Methylformiat, Essigsäuremethylester oder Essigsäureethylester oder Alkohole wie beispielsweise Methanol, Ethanol, n-Propanol, iso-Propanol und tert.-Butanol sowie beliebige Mischungen solcher Lösungsmittel.

Die Verbindungen der Formel (I) sind von der Erfindung ebenfalls umfasst.

Für die Vorzugsbereiche gelten die vorstehend gemachten Angaben analog.

Als Einzelverbindungen der Formel (I) seien genannt:
2-Hydroxy-5-fluor-phenylacetonitril, 2-Hydroxy-4,5-difluor-phenylacetonitril, 2-Hydroxy-5-trifluormethoxy-phenylacetonitril, 6-Hydroxy-2,3,4-trifluor-phenyiacetonitril und 2-Hydroxy-4-trifluormethyl-phenylacetonitril.

Gegebenenfalls kann in einem Schritt d) die Umsetzung von Verbindungen der Formel (I) mit Verbindungen der Formeln (VIIa) oder (VIIb)

R⁵CO-X (VIIa)

R⁶-Y (VIIb)

wobei in Formel (VIIa)
- R⁵: für Wasserstoff, C₁-C₁₂-Alkyl, C₂-C₁₂-Alkenyl, C₅-C₁₄-Aryl, C₆-C₁₅-Arylalkyl, O(C₁-C₁₂-Alkyl), O(C₅-C₁₄-Aryl), O(C₆-C₁₅-Arylalkyl), O(C₂-C₁₂-Alkenyl), NH(C₁-C₁₂-Alkyl), NH(C₅-C₁₄-Aryl), NH(C₆-C₁₅-Arylalkyl), N(C₁-C₁₂-Alkyl)₂, N(C₅-C₁₄-Aryl)₂ oder N(C₆-C₁₅-Arylalkyl)₂, bevorzugt für C₁-C₁₂-Alkyl und besonders bevorzugt für Methyl steht und
- X: für OCOR⁵, Fluor, Chlor, Brom oder Iod steht und
wobei in Formel (VIIb)
- R⁶: für C₁-C₁₂-Alkyl, C₅-C₁₄-Aryl oder C₆-C₁₅-Arylalkyl, bevorzugt für C₁-C₁₂-Alkyl und besonders bevorzugt für Methyl steht und
- Y: für O₃SR⁷, Chlor, Brom oder Iod steht, wobei R⁷ für C₁-C₁₂-Alkyl, C₅-C₁₄-Aryl oder C₁-C₁₂-Fluoralkyl steht,
zu Verbindungen der Formel (VIII) erfolgen, 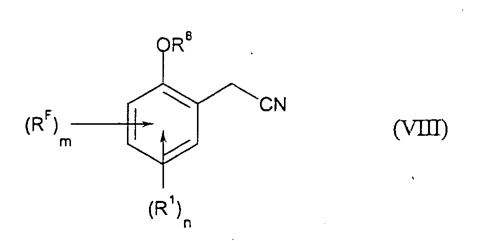 in der
- R⁸: für R⁵CO oder R⁶ mit der vorstehend angegebenen Bedeutung steht.

Diese Acylierung oder Alkylierungen können in an sich bekannter Weise zum Beispiel in Gegenwart einer Base durchgeführt werden.

Verbindungen der Formel (VIIIa), 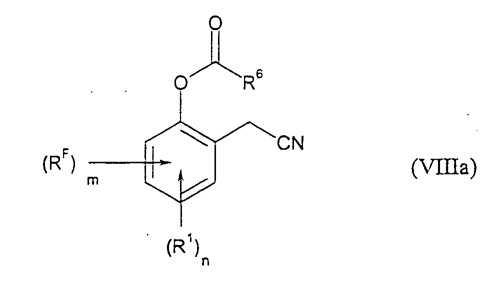 in der R¹, R⁶ und R^{F}, sowie m und n die vorstehend angegebenen Bedeutungen und Vorzugsbereiche besitzen, und die eine Teilmenge der Verbindungen der Formel (VIII) sind, sind von der Erfindung ebenfalls umfasst. Die oben genannten Vorzugsbereiche gelten dabei entsprechend.

Von der Erfindung sind weiterhin auch Verfahren umfasst, die zumindest einen der Schritte a) zur Herstellung von Verbindungen der Formel (IV), b) zur Herstellung von Verbindungen der Formel (VI) und c) zur Herstellung von Verbindungen der Formel (I) umfassen. Die vorstehend angegebenen Vorzugsbereiche und Parameter gelten dabei analog.

Die erfindungsgemäß erhältlichen Verbindungen der Formeln (I) und (VIII) eignen sich insbesondere in einem Verfahren zur Herstellung von Wirkstoffen wie beispielsweise von Wirkstoffen für Arzneimitteln. Bevorzugte Wirkstoffe für Arzneimittel sind solche, die zur Behandlung von Herz-Kreislauf-Erkrankungen eingesetzt werden. Insbesondere sind das diejenigen die in der WO-A 2/19780 beschrieben sind.

Ein wesentlicher Vorteil der Erfindung ist, dass die Verbindungen der Formel (I) in einfacher Weise aus gut verfügbaren Edukten hergestellt werden können. Weiterhin stellen die erfindungsgemäßen Verbindungen der Formeln (I), (IV), (VI) und (VIII) wertvolle Ausgangsprodukte für die Herstellung von Wirkstoffen insbesondere für Arzneimittel dar.

### Beispiele

### Beispiel 1

### Herstellung von 4,5-Difluor-2-hydroxy-N,N-dimethylbenzylamin

400 g 3,4-Difluorphenol werden in 408 ml 40 %iger wässriger Dimethylamin-Lösung vorgelegt und auf 0°C gekühlt. Bei 0-5°C werden 276 ml 37 %ige wässrige Formaldehyd-Lösung innerhalb von 60 min. zugetropft. Es wird noch 2 Stunden bei 5-10°C gehalten und anschließend 20 Std. bei Raumtemperatur gerührt. Der Ansatz wird mit 600 ml Wasser versetzt. Man trennt die organische Phase ab, extrahiert die wässrige Phase zweimal mit Dichlormethan, trocknet die vereinigten organischen Phasen und destilliert das Lösungsmittel im Vakuum ab. Das Rohprodukt wird anschließend im Vakuum fraktioniert destilliert. Man erhält 395 g (65 % der Theorie) einer farblosen Flüssigkeit mit einem Siedepunkt von 93°C bei 16 mbar.

Das ¹H-NMR-Spektrum enthielt folgende charakteristischen Absorptionen (CDCl₃, δ/ppm):
11.32 (s, 1 H, OH); 7.77, 7.60 (2 m, 2 H, H-3, H-6); 3.57 (s, 2 H, CH₂); 2.32 (s, 6 H, N(CH₃)₂).

Mittels GC-MS erhält man folgendes Spektrum (EI, 70eV, I/%): 187 (100, M⁺); 143 (36, (M-N(CH₃)₂)⁺).

### Beispiel 2

### Herstellung von 2-Hydroxy-5-(trifluormethoxy)-N,N-dimethylbenzylamin

130 g 4-Trifluormethoxyphenol werden in 97 ml 40 %iger wässriger Dimethylamin-Lösung vorgelegt und auf 3°C gekühlt. Bei 0-5°C werden 66 ml 37 %ige wässrige Formaldehyd-Lösung innerhalb von 45min. zugetropft. Es wird noch 2 Stunden bei 5-10°C gehalten und anschließend 19 Std. bei Raumtemperatur gerührt. Es wird erneut auf 0°C abgekühlt, 4,9 ml 40 %ige wässrige Dimethylamin-Lösung zugesetzt und anschließend 3,3 ml 37 %ige wässrige Formaldehyd-Lösung zugetropft. Man lässt 3 Stunden bei Raumtemperatur nachrühren. Der Ansatz wird mit 100 ml Wasser versetzt. Man trennt die organische Phase ab, extrahiert die wässrige Phase zweimal mit Dichlormethan, trocknet die vereinigten organischen Phasen und destilliert das Lösungsmittel im Vakuum ab. Das Rohprodukt wird anschließend im Vakuum fraktioniert destilliert. Man erhält 118 g (69 % der Theorie) einer hellgelben Flüssigkeit mit einem Siedepunkt von 110-112°C bei 18 mbar.

Das ¹H-NMR-Spektrum enthielt folgende charakteristischen Absorptionen (CDCl₃, δ/ppm): 11.08 (bs, 1 H, OH); 7.03 (dd, 1 H, J _{H4-H3}= 8.9 Hz, J _{H-4-H6}= 2.4 Hz, H-4); 6.84 (d, 1H, J_{H-6, H-4}= 2.3 Hz, H-6); 6.80 (d, 1 H, J_{H3-H4} = 8.8 Hz, H-3); 3.62 (s, 2 H, CH₂); 2.32 (s, 3 H, CH₃).

Mittels GC-MS erhält man folgendes Spektrum (EI, 70eV, I/%): 235 (100, M⁺); 191 (19, (M-N(CH₃)₂)⁺).

### Beispiel 3

### Herstellung von 6-Hydroxy-2,3,4-trifluor-N,N-dimethylbenzylamin

400 g 3,4,5-Trifluorphenol werden in 359 ml 40 %iger wässriger Dimethylamin-Lösung vorgelegt und auf 0°C gekühlt. Bei 0-5°C werden 243 ml 37 %ige wässrige Formaldehyd-Lösung innerhalb von 90min. zugetropft. Anschließend wird 20 Std. bei Raumtemperatur gerührt. Der Ansatz wird mit 600 ml Wasser und 500 ml Dichlormethan versetzt. Man trennt die organische Phase ab, extrahiert die wässrige Phase einmal mit Dichlormethan, trocknet die vereinigten organischen Phasen und destilliert das Lösungsmittel im Vakuum ab. Das Rohprodukt wird in 500ml Wasser aufgenommen und unter Kühlung im Eisbad mit verdünnter Salzsäure auf pH 1-2 eingestellt. Die saure Lösung wird einmal mit Dichlormethan extrahiert und anschließend unter Kühlung im Eisbad mit verdünnter Natronlauge auf pH 8-9 gestellt. Die alkalische Reaktionslösung wird dreimal mit Dichlormethan extrahiert, die vereinigten organischen Phasen werden einmal mit Wasser gewaschen, getrocknet und das Lösungsmittel im Vakuum abdestilliert. Das Rohprodukt wird anschließend im Vakuum fraktioniert destilliert. Man erhält 305 g (55 % der Theorie) einer hellgelben Flüssigkeit mit einem Siedepunkt von 96°C bei 10 mbar.

Das ¹H-NMR-Spektrum enthielt folgende charakteristischen Absorptionen (CDCl₃, δ/ppm): 11.70 (s, 1 H, OH); 6.41 (m, 1 H, H-5); 3.71 (s, 2 H, CH₂); 2.37 (s, 6 H, N(CH₃)₂).

### Beispiel 4

### Herstellung von 2-Hydroxy-4-(trifluormethyl)-N,N-dimethylbenzylamin

195 g 3-Trifluormethylphenol werden in 160 ml 40 %iger wässriger Dimethylamin-Lösung vorgelegt und auf 15°C gekühlt. Bei 155°C werden 108 ml 37 %ige wässrige Formaldehyd-Lösung innerhalb von 40 min. zugetropft. Anschließend wird 20 Std. bei Raumtemperatur gerührt. Man kühlt erneut auf 15°C ab, versetzt mit 8 ml 40 %iger wässriger Dimethylamin-Lösung und tropft 5,5 ml 37 %ige wässrige Formaldehyd-Lösung zu. Anschließend wird 4,5 Stunden bei Raumtemperatur nachgerührt. Man kühlt erneut auf 15°C ab, versetzt mit 32 ml 40 %iger wässriger Dimethylamin-Lösung und tropft 21 ml 37 %ige wässrige Formaldehyd-Lösung zu. Anschließend wird 17 Stunden bei Raumtemperatur nachgerührt. Der Ansatz wird mit 150 ml Wasser versetzt. Man trennt die organische Phase ab, extrahiert die wässrige Phase zweimal mit Dichlormethan, wäscht die vereinigten organischen Phasen einmal mit Wasser und destilliert das Lösungsmittel im Vakuum ab. Das Rohprodukt wird in 250ml Wasser aufgenommen und unter Kühlung im Eisbad mit verdünnter Salzsäure auf pH 1-2 eingestellt. Die saure Lösung wird einmal mit Dichlormethan extrahiert und anschließend unter Kühlung im Eisbad mit verdünnter Natronlauge auf pH 11 gestellt. Die alkalische Reaktionslösung wird zweimal mit Dichlormethan extrahiert, die vereinigten organischen Phasen werden einmal mit Wasser gewaschen, getrocknet und das Lösungsmittel im Vakuum abdestilliert. Das Rohprodukt wird anschließend im Vakuum fraktioniert destilliert. Man erhält 186 g (71% der Theorie) einer hellgelben Flüssigkeit mit einem Siedepunkt von 91°C bei 14 mbar.

Das ¹H-NMR-Spektrum enthielt folgende charakteristischen Absorptionen (CDCl₃, δ/ppm): 7.08-6.98 (m, 3 H, H-3, H-5, H-6); 3.67 (s, 2 H, CH₂); 2.32 (s, 6 H, N(CH₃)₂).

### Beispiel 5

### Herstellung von 2-Acetory-4,5-difluorbenzylacetat

200 g 4,5-Difluor-2-hydroxy-N,N-dimethylbenzylamin werden in 350 ml Toluol gelöst und auf Rückfluss erhitzt. Es werden 252 ml Essigsäureanhydrid zugetropft und der Ansatz bis zum vollständigen Umsatz bei Rückfluss gerührt. Man lässt auf 70°C abkühlen, tropft 100 ml Methanol zu und rührt 1 Std. bei 70°C. Nach dem Abkühlen auf Raumtemperatur wäscht man zweimal mit 5 %iger Salzsäure und einmal mit Wasser. Das Lösungsmittel wird im Vakuum abdestilliert. Das Rohprodukt wird im Vakuum fraktioniert. Man erhält 127 g (48 % der Theorie) 2-Acetoxy-4,5-difluorbenzylacetat als farblose Flüssigkeit mit einem Siedepunkt von 119°C bei 2,6 mbar.

Das ¹H-NMR-Spektrum enthielt folgende charakteristischen Absorptionen (CDCl₃, δ/ppm): 7.28,7.00 (2 x m, 2 H, H-3, H-6); 5.00 (s, 2 H, CH₂); 2.32, 2.08 (2 x s, 6 H, COCH₃).

Mittels GC-MS erhält man folgendes Spektrum (EI, 70eV, I/%): 244 (10, M⁺); 202 (100, (M-CH₂CO)⁺); 160 (23, (M -2 CH₂CO)⁺); 142 (92, (202-HOAc)⁺); 114 (45, (142-CO)⁺).

### Beispiel 6

### Herstellung von 2-Acetoxy-5-(trifluormethoxy)benzylacetat

160 g 2-Hydroxy-5-(trifluormethoxy)-N,N-dimethylbenzylamin werden in 400ml Toluol gelöst und auf Rückfluss erhitzt. Es werden 160 ml Essigsäureanhydrid zugetropft und der Ansatz bis zum vollständigen Umsatz bei Rückfluss gerührt. Man lässt auf 70°C abkühlen, tropft 100 ml Methanol zu und rührt 1 Std. bei 70°C. Nach dem Abkühlen auf Raumtemperatur wäscht man zweimal mit 5 %iger Salzsäure und einmal mit Wasser. Das Lösungsmittel wird im Vakuum abdestilliert. Das Rohprodukt wird im Vakuum fraktioniert. Man erhält 175 g (87% der Theorie) 2-Acetoxy-5-(trifluormethoxy)benzylacetat als farblose Flüssigkeit mit einem Siedepunkt von 107-110°C bei 2,9 mbar.

Das ¹H-NMR-Spektrum enthielt folgende charakteristischen Absorptionen (CDCl₃, δ/ppm): 7.32 (d, 1 H, J_{H6-H4}=2.25 Hz, H-6); 7.21 (dd, 1 H, J_{H4-H6}=2.13 Hz, J_{H-4-H3}=8.88 Hz, H-4); 7.13 (d, 1 H, J_{H3-H-4}=8.88 Hz, H-3); 5.06 (2, 2 H, CH₂); 2.32, 2.09 (2 s, 6 H, 2x COCH₃).

Mittels GC-MS erhält man folgendes Spektrum (EI, 70eV, I/%): 292 (2, M+); 250 (54, (M-CH₂CO)+); 190 (100, (M-CH₂CO-HOAc)+); 162 (46, (190-CO)⁺).

### Beispiel 7

### Herstellung von 6-Acetoxy-2,3,4-trifluorbenzylacetat

170 g 6-Hydroxy-2,3,4-trifluor-N,N-dimethylbenzylamin werden in 425 ml Toluol gelöst und auf Rückfluss erhitzt. Es werden 195 ml Essigsäureanhydrid zugetropft und der Ansatz bis zum vollständigen Umsatz bei Rückfluss gerührt. Man lässt auf 70°C abkühlen, tropft 100 ml Methanol zu und rührt 1 Std. bei 70°C. Nach dem Abkühlen auf Raumtemperatur wäscht man zweimal mit 5 %iger Salzsäure und einmal mit Wasser. Das Lösungsmittel wird im Vakuum abdestilliert. Der. Rückstand wird in 1000 ml Methyl-tert.-Butylether eingetropft, der Niederschlag abfiltriert und das Filtrat im Vakuum eingeengt. Das Rohprodukt wird im Vakuum fraktioniert. Man erhält 109 g (50 % der Theorie) 6-Acetoxy-2,3,4-trifluorbenzylacetat als farblose Flüssigkeit mit einem Siedepunkt von 94°C bei 1,8 mbar.

Das ¹H-NMR-Spektrum enthielt folgende charakteristischen Absorptionen (CDCl₃, δ/ppm): 6.85 (m, 1 H, H-5); 5.12 (s, 2 H, CH2); 2.33, 2.04 (2 x s, 6 H, 2 x COCH₃).

Mittels GC-MS erhält man folgendes Spektrum (EI, 70eV, I/%): 262 (2, M⁺), 220 (55, (M-CH₂CO)⁺); 160 (100, (M-CH₂CO-HOAc)⁺); 132 (37, (160-CO)⁺).

### Beispiel 8

### Herstellung von 2-Acetoxy-4-trifluormethylbenzylacetat

100 g 2-Hydroxy-4-trifluormethyl-N,N-dimethylbenzylamin werden in 180 ml Toluol gelöst und auf Rückfluss erhitzt. Es werden 108 ml Essigsäureanhydrid zugetropft und der Ansatz bis zum vollständigen Umsatz bei Rückfluss gerührt. Man lässt auf 70°C abkühlen, tropft 100 ml Methanol zu und rührt 1 Std. bei 70°C. Nach dem Abkühlen auf Raumtemperatur wäscht man zweimal mit 5 %iger Salzsäure und einmal mit Wasser. Das Lösungsmittel wird im Vakuum abdestilliert. Das Rohprodukt wird im Vakuum fraktioniert. Man erhält 113 g (88 % der Theorie) 2-Acetoxy-4-trifluormethylbenzylacetat als farblose Flüssigkeit mit einem Siedepunkt von 113-115°C bei 4,1 mbar.

Das ¹H-NMR-Spektrum enthielt folgende charakteristischen Absorptionen (CDCl₃, δ/ppm): 7.55 (m, 2 H, H-5, H-6); 7.40 (s, 1 H, H-6); 5.10 (s, 2 H, CH₂); 2.34, 2.10 (2 x s, 6 H, 2 x COCH₃).

Mittels GC-MS erhält man folgendes Spektrum (EI, 70eV, I/%): 276 (3, M⁺); 257 (7, (M-F)⁺); 234 (84, (M-CH₂CO)⁺); 192 (32, (M-2 CH₂CO)⁺); 174 (100, (M-CH₂CO-HOAc)⁺); 146 (57, (174-CO)⁺).

### Beispiel 9

### Herstellung von 2-Hydroxy-5-fluor-phenyl-acetonitril

151,2 g 2-Hydroxy-5-fluorbenzylalkohol-diacetat (0,66 mol) wurden in 500 ml Methanol und 250 ml Ethylacetat gelöst. Dann wurden 81,1 g Natriumcyanid (1,65 mol, 2,5 eq) zugesetzt. Dabei stieg die Temperatur auf 44°C an. Der Ansatz wurde noch 2 h bei 44°C gehalten, dann wurde über Nacht bei Raumtemperatur gerührt. Anschließend wurde auf 50°C geheizt und 5,5 h nachgerührt.

Zur Aufarbeitung wurden je 700 ml gesättigte NaCl-Lösung und Ethylacetat sowie 300 ml Wasser zugesetzt, der Ansatz gerührt und die Phasen getrennt. Die wässrige Phase wurde noch 2 x mit je 250 ml Ethylacetat nachextrahiert, die vereinigten org. Phasen wurden mit 2 x 500 ml Wasser und mit 1 x 500 ml 1N-Salzsäure gewaschen. Nach Abdestillieren den Lösungsmittels im Vakuum wurde ein schwarzbraunes Öl erhalten, das beim Abkühlen kristallisierte. Es wurden 98,1 g Rohprodukt mit einem Gehalt von 77,2 Gew.-% (HPLC-ESTD; 83 Fl.-%) erhalten (0,50 mol), entsprechend einer Ausbeute von 76 % der Theorie.
¹H-NMR (400 MHz, DMSO): δ= 3,8 ppm (s, -CH₂OH); 6,85 ppm (1 Hₐᵣ), 7,0 ppm (1 Hₐᵣ), 7,1 ppm (1 Hₐᵣ); LC-MS: [M]⁺ =151; 124 (-HCN); 96.

## Patentansprüche

1. Verfahren zur Herstellung von Verbindungen der Formel (I), 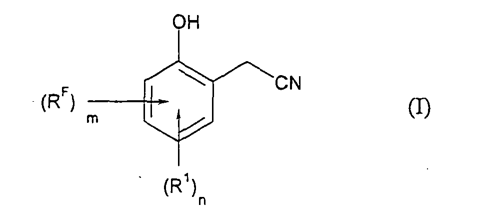 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht,
**dadurch gekennzeichnet, dass**
a) Verbindungen der Formel (II) 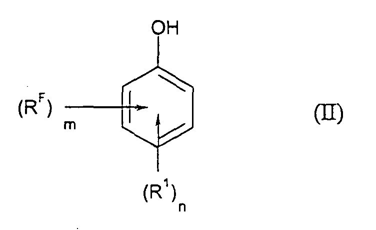 in der R¹ und R^{F}, sowie n und m die vorstehend genannte Bedeutung besitzen,
• in Gegenwart von Formaldehyd und
• in Gegenwart von sekundären Aminen der Formel (III)
HNR³R⁴ (III)
in der R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht
zu Verbindungen der Formel (IV) umgesetzt werden, 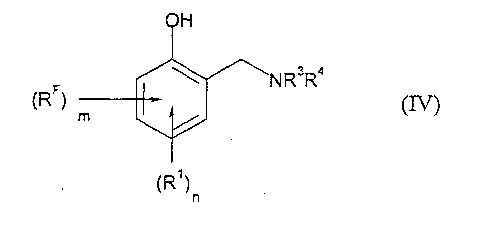 in der R¹, R³, R⁴ und R^{F} sowie m und n die vorstehend genannte Bedeutung besitzen und
b) die Verbindungen der Formel (IV) durch Umsetzung mit Verbindungen der Formel (V)
R⁵CO-O-OCR⁵ (V)
in der die Reste R⁵ jeweils unabhängig voneinander für Wasserstoff, C₁-C₁₂-Alkyl, C₂-C₁₂-Alkenyl, C₅-C₁₄-Aryl oder C₆-C₁₅-Arylalkyl stehen,
in Verbindungen der Formel (VI) überführt werden 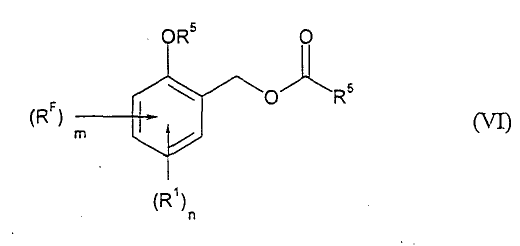 und
c) die Verbindungen der Formel (VI) durch Umsetzung mit Cyanid in Verbindungen der Formel (I) überführt werden.

2. Verfahren nach Anspruch 1, **dadurch gekennzeichnet, dass** R¹ jeweils unabhängig für C₁-C₄-Alkyl, freies oder geschütztes Formyl oder Chlor steht.

3. Verfahren nach mindestens einem der Ansprüche 1 bis 2, **dadurch gekennzeichnet, dass** n für 0 oder 1 steht.

4. Verfahren nach mindestens einem der Ansprüche 1 bis 3, **dadurch gekennzeichnet, dass** R^{F} für Fluor, C₁-C₄-Fluoralkyl, -O(C₁-C₄-Fluoralkyl) oder -S(C₁-C₄-Fluoralkyl) steht.

5. Verfahren nach mindestens einem der Ansprüche 1 bis 4, **dadurch gekennzeichnet, dass** R³ und R⁴ jeweils für einen identischen C₁-C₈-Alkylrest stehen.

6. Verfahren nach mindestens einem der Ansprüche 1 bis 5, **dadurch gekennzeichnet, dass** R⁵ jeweils identisch für Wasserstoff, C₁-C₁₂-Alkyl, C₂-C₁₂-Alkenyl, C₅-C₁₄-Aryl oder C₆-C₁₅-Arylalkyl steht.

7. Verfahren nach mindestens einem der Ansprüche 1 bis 6, **dadurch gekennzeichnet, dass** im Schritt a) das molare Verhältnis von Formaldehyd zu Verbindungen der Formel (II) 0,8 bis 10 beträgt.

8. Verfahren nach mindestens einem der Ansprüche 1 bis 7, **dadurch gekennzeichnet, dass** im Schritt a) das molare Verhältnis von sekundären Aminen der Formel (III) zu Verbindungen der Formel (II) 0,8 bis 10 beträgt.

9. Verfahren nach mindestens einem der Ansprüche 1 bis 8, **dadurch gekennzeichnet, dass** im Schritt b) das molare Verhältnis von Verbindungen der Formel (V) zu Verbindungen der Formel (IV) 1,5 bis 10 beträgt.

10. Verfahren nach mindestens einem der Ansprüche 1 bis 9, **dadurch gekennzeichnet, dass** im Schritt c) Alkalimetallcyanide eingesetzt werden.

11. Verfahren nach mindestens einem der Ansprüche 1 bis 10, **dadurch gekennzeichnet, dass** in einem weiteren Schritt d) die Verbindungen der Formel (I) mit Verbindungen der Formeln (VIIa) oder (VIIb)
R⁵CO-X (VIIa)
R⁶-Y (VIIb)
wobei in Formel (VIIa)
R⁵ für Wasserstoff, C₁-C₁₂-Alkyl, C₂-C₁₂-Alkenyl, C₅-C₁₄-Aryl, C₆-C₁₅-Arylalkyl, O(C₁-C₁₂-Alkyl), O(C₅-C₁₄-Aryl), O(C₆-C₁₅-Arylalkyl), O(C₂-C₁₂-Alkenyl), NH(C₁-C₁₂-Alkyl), NH(C₅-C₁₄-Aryl), NH(C₆-C₁₅-Arylalkyl), N(C₁-C₁₂-Alkyl)₂, N(C₅-C₁₄-Aryl)₂ oder N(C₆-C₁₅-Arylalkyl)₂, bevorzugt für C₁-C₁₂-Alkyl und besonders bevorzugt für Methyl steht und
X für OCOR⁵, Fluor, Chlor, Brom oder Iod steht und
wobei in Formel (VIIb)
R⁶ für C₁-C₁₂-Alkyl, C₅-C₁₄-Aryl oder C₆-C₁₅-Arylalkyl steht und
Y für O₃SR⁷, Chlor, Brom oder Iod steht, wobei R⁷ für C₁-C₁₂-Alkyl, C₅-C₁₄-Aryl oder C₁-C₁₂-Fluoralkyl steht,
zu Verbindungen der Formel (VIII) umgesetzt werden, 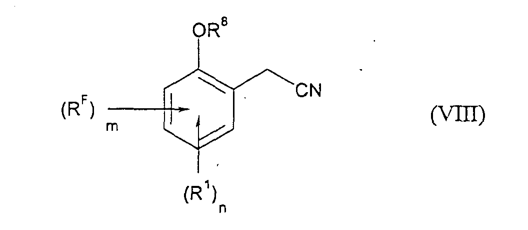 in der
R⁸ für R⁵CO oder R⁶ mit der vorstehend angegebenen Bedeutung steht und R¹, R^{F}, m und n die unter der Formel (I) genannte Bedeutung besitzen.

12. Verfahren zur Herstellung von Verbindungen der Formel (I), 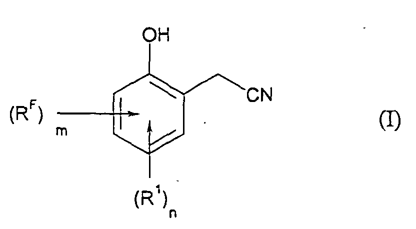 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂, oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht,
**dadurch gekennzeichnet, dass** Verbindungen der Formel (VI) 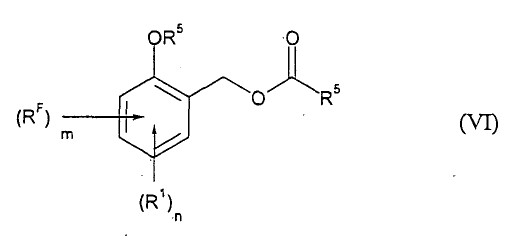 in der R¹, R^{F}, m und n die unter der Formel (I) angegebene Bedeutung besitzen und
die Reste R⁵ jeweils unabhängig voneinander für Wasserstoff, C₁-C₁₂-Alkyl, C₂-C₁₂-Alkenyl, C₅-C₁₄-Aryl oder C₆-C₁₅-Arylalkyl stehen durch Umsetzung mit Cyanid in Verbindungen der Formel (I) überführt werden.

13. Verfahren zur Herstellung von Verbindungen der Formel (VI), 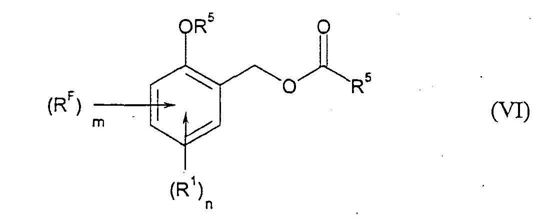 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht und
R⁵ jeweils unabhängig voneinander für Wasserstoff, C₁-C₁₂-Alkyl, C₂-C₁₂-Alkenyl, C₅-C₁₄-Aryl oder C₆-C₁₅-Arylalkyl steht,
**dadurch gekennzeichnet, dass** Verbindungen der Formel (IV), 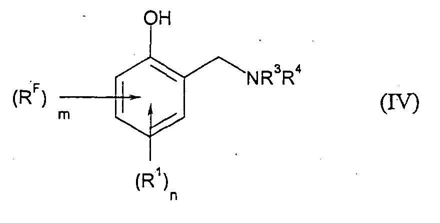 in der R¹ und R^{F} , sowie m und n die vorstehend genannte Bedeutung besitzen und
R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht mit Verbindungen der Formel (V) umgesetzt werden
R⁵CO-O-OCR⁵ (V),
in der die Reste R⁵ die vorstehend genannte Bedeutung besitzen.

14. Verfahren zur Herstellung von Verbindungen der Formel (IV), 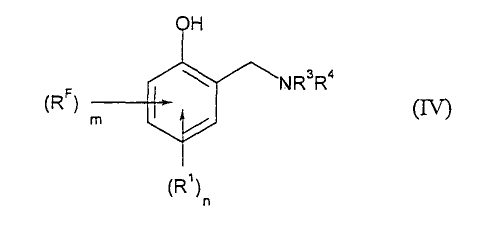 in der
R¹ jeweils unabhängig für C₁-C₁₂-Alkyl, freies oder geschütztes Formyl, Chlor oder Brom oder für Reste der Formeln (Ila) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht und
R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht
**dadurch gekennzeichnet, dass** Verbindungen der Formel (II) 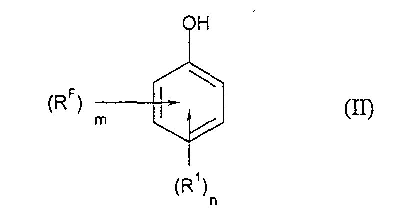 in der R¹ und R^{F}, sowie n und m die vorstehend genannte Bedeutung besitzen,
• in Gegenwart von Formaldehyd und
• in Gegenwart von sekundären Aminen der Formel (III)
HNR³R⁴ (III)
in der R³ und R⁴ die vorstehend genannte Bedeutung besitzen,
umgesetzt werden.

15. Verbindungen der Formel (I), 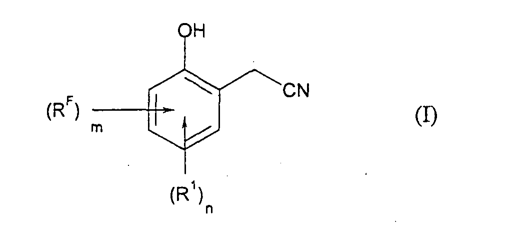 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht.

16. 2-Hydroxy-5-fluor-phenylacetonitril, 2-Hydroxy-4,5-difluor-phenylacetonitril, 2-Hydroxy-5-trifluormethoxy-phenylacetonitril, 6-Hydroxy-2,3,4-trifluor-phenylacetonitril und 2-Hydroxy-4-trifluormethyl-phenylacetonitril.

17. Verbindungen der Formel (IV), 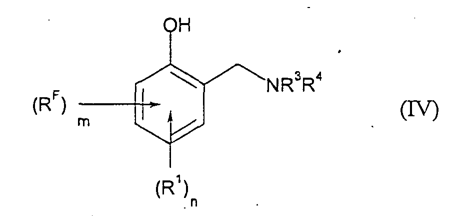 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht und
R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht
wobei jedoch 2-Hydroxy-5-fluor-N,N-dimethylbenzylamin ausgenommen ist.

18. 4,5-Difluor-2-hydroxy-N,N-dimethylbenzylamin, 2-Hydroxy-5-(trifluormethoxy)-N,N-dimethylbenzylamin, 6-Hydroxy-2,3,4-trifluor-N,N-dimethylbenzylamin und 2-Hydroxy-4-(trifluormethyl)-N,N-dimethylbenzylamin.

19. Verbindungen der Formel (VI), 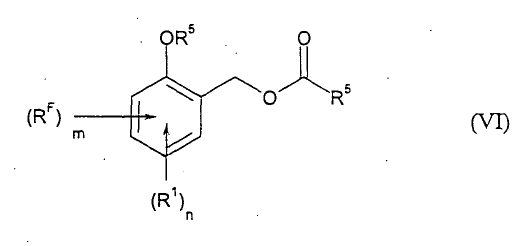 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₆-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht und
R⁵ jeweils unabhängig voneinander für Wasserstoff, C₁-C₁₂-Alkyl, C₂-C₁₂-Alkenyl, C₅-C₁₄-Aryl oder C₆-C₁₅-Arylalkyl steht, wobei jedoch 2-Acetoxy-5-fluorbenzylacetat ausgenommen ist.

20. 2-Acetoxy-4,5-difluorbenzylacetat, 2-Acetoxy-5-(trifluormethoxy)benzylacetat, 6-Acetoxy-2,3,4-trifluorbenzylacetat und 2-Acetoxy-4-trifluormethylbenzylacetat,

21. Verbindungen der Formel (VIIIa), 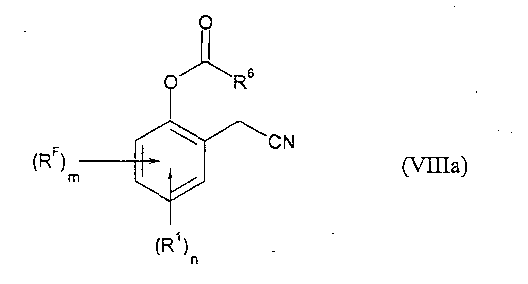 in der
R¹ jeweils unabhängig für C₁-C₁₂-Alkyl, freies oder geschütztes Formyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 4-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht und
R⁶ für C₁-C₁₂-Alkyl, C₅-C₁₄-Aryl oder C₆-C₁₅-Arylalkyl steht.

22. Verwendung von Verbindungen gemäß mindestens einem der Ansprüche 15 bis 21 oder Verbindungen, die nach mindestens einem der Ansprüche 1 bis 14 hergestellt wurden zur Herstellung von Wirkstoffen für Arzneimittel.

23. Verwendung nach Anspruch 22, **dadurch gekennzeichnet, dass** die Wirkstoffe für Arzneimittel zur Behandlung von Herz-Kreislaufbeschwerden oder -erkrankungen eingesetzt werden.
